(19)

Europäisches Patentamt
European Patent Office
Office européen des brevets

(11) **EP 3 586 739 A1**

(12) **EUROPEAN PATENT APPLICATION**

(43) Date of publication:
**01.01.2020 Bulletin 2020/01**

(21) Application number: **18180023.6**

(22) Date of filing: **27.06.2018**

(51) Int Cl.:
*A61B 5/08* (2006.01)      *G01N 33/00* (2006.01)
*A61B 5/145* (2006.01)     *F24F 11/00* (2018.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **TIEMANN, Christian Andreas**
  **5656 AE Eindhoven (NL)**
• **WOUTERS, Cornelis Bernardus Aloysius**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **UNOBTRUSIVE HEALTH ANALYSIS**

(57)   A method of estimating a ventilation rate in a room, the method comprising receiving a signal indicative of a concentration of at least one Volatile organic compound (VOC(t)) inside the room; processing the signal to detect at least one increase from an initial level (VOC(t0)) of the concentration of the at least one volatile organic compound; detect a corresponding maximum level of the concentration from the initial level; detecting when the concentration of the volatile organic compound returns to the initial level or a steady state level (VOCss) from the corresponding maximum level; and determining the ventilation rate (k) based on the time (t) taken by the concentration of the at least one Volatile organic compound to reach to the initial level from the corresponding maximum level.

FIG. 2

EP 3 586 739 A1

**Description**

FIELD OF THE INVENTION

The present invention relates to the field of health analysis, in particular unobtrusive health analysis.

BACKGROUND OF THE INVENTION

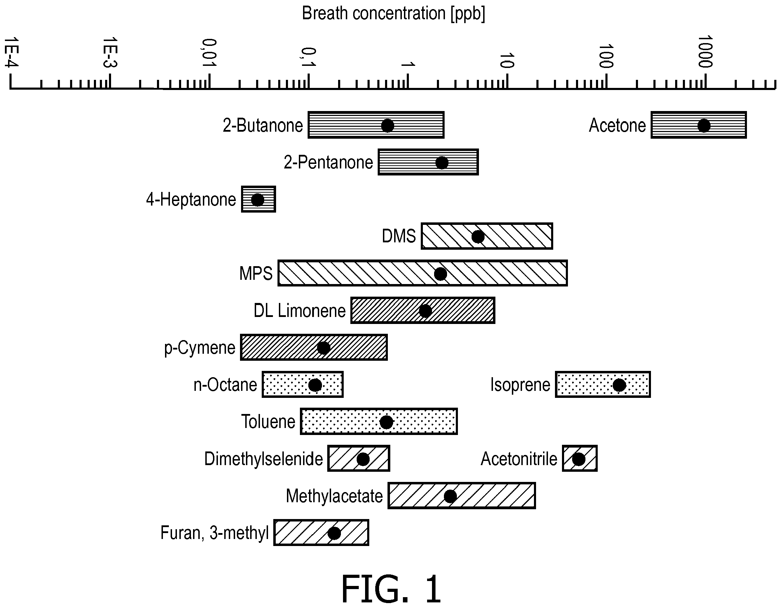

Exhaled breath analysis is becoming an increasingly important non-invasive diagnostic method that can be used in the evaluation of health status and disease types. Thousands of molecules are emitted into the air when we breathe. A breath sample is typically composed of inorganic gases (e.g., NO, $CO_2$, and CO), Volatile Organic Compounds (VOCs) (e.g., isoprene, ethane, pentane, and acetone), and other non-volatile substances (e.g., isoprostanes, peroxynitrite, cytokines, and nitrogen). Fig. 1 shows an overview of the most prominent VOCs present in human breath (Agapiou et al. Trace detection of endogenous human volatile organic compounds for search, rescue and emergency applications, Trends in Analytical Chemistry 66 (2015) 158-175). Detailed analysis of breath components can provide information about physiological processes that take place in the body, and hereby provide information of the current health status. For instance, a VOC of frequent occurrence is acetone which is a ketone body being produced by the liver from fatty acids during periods of low food intake. Acetone is also known to be elevated in diabetic patients. Furthermore, breath analyses have been successfully employed to identify conditions like lung disease, oxidative stress, gastrointestinal disease, and metabolic disorders.

Although breath analysis is non-invasive in nature, which is ideal in a hospital setting to replace certain blood tests, there are limitations to use it in a home setting, for instance for regular monitoring. Breath analysis could be perceived as obtrusive as it still requires a device to capture a breath sample (e.g., a mask or tube). This might not be comfortable and it is not very attractive to perform tests on a regular basis. Next to this, it might also be costly as such a device probably requires a disposable part for hygiene reasons.

SUMMARY OF THE INVENTION

It is an object of the present invention to provide an unobtrusive way to analyze health of the person by means of detecting certain compounds of interest and their release rate of these compounds of interest. These compounds of interest are released by a person in a room, for instance by means of breath, flatulence, through skin, etc. In various embodiments of the invention, the person may be a baby, an elderly or any other human being. The methods described below are best suited for a single person located in a room for a prolonged period of time, e.g., during sleep.

The invention proposes to estimate the rate of exhaled compounds of interest in an indirect way by measuring the concentration of these compounds in the air of the room the person is located in, and by estimating the ventilation rate of the room. The air concentration of a compound is determined by the inflow of the compound into the room (e.g., via human breathing) and the outflow of the compound to adjacent areas via doors and windows. The ventilation rate helps to determine the release rate of the compound of interest, which thereby helps to understand the amount of compound of interest being released in a period of time, which further then facilitates determining the health of the person.

In a first aspect of the present disclosure a method of estimating a ventilation rate in a room is provided. The method comprising:

receiving a signal indicative of a concentration of at least one Volatile Organic Compound (VOC(t)) inside the room; processing the signal to

detect at least one increase from an initial level (VOC(t0)) of the concentration of the at least one volatile organic compound; detect a corresponding maximum level of the concentration from the initial level; detect when the concentration of the volatile organic compound returns to the initial level or a steady state level (VOCss) from the corresponding maximum level; and determine the ventilation rate (k) based on the time (t) taken by the concentration of the at least one Volatile organic compound to reach to the initial level from the corresponding maximum level.

Advantageously a method of estimating a ventilation rate is provided which makes use of only a Volatile Organic Compound (VOC) sensor to understand the temporary increases of VOCs, mostly gases, in the room and subsequent diffusion, which is effected by the ventilation rate of the room. Since the VOCs used for determining ventilation are temporary in nature, such as VOCs released by flatulence, it is easy to distinguish the abrupt increases in the VOC signal as these VOCs also subside (diffuse) within a few minutes, due to ventilation.

In a further aspect of the present disclosure, the method further includes estimating release rate of a compound of interest, wherein the release rate is of the person, wherein estimating the release rate further comprises:

- receiving a signal indicative of a first concentration of the compound of interest inside the room, wherein the compound of interest is being released by the person;
- receiving a signal indicative of a second concentration of the compound of interest outside the room; and
- calculating the release rate of the compound of interest based on the first concentration, second concentration and the ventilation rate.

Advantageously, the method steps above helps in determination of the release rate of the compound of interest in a non-obtrusive way. The person concerned is not disturbed in any sense and also is not asked to wear any mask or to breathe in a device to understand the release rate of certain compound of interest in his breath (for instance).

In another aspect of the present disclosure, an apparatus for estimating a ventilation rate in a room is provided, the apparatus includes:

a. a signal interface configured for receiving a signal indicative of a concentration of at least one Volatile organic compound inside the room, wherein the signal is received from a sensor configured for detecting the at least one Volatile organic compound;

b. a processing unit configured for:

i. detecting at least one increase from an initial level of the concentration of the at least one volatile organic compound;

ii. detecting a corresponding maximum level of the concentration from the initial level;

iii. detecting when the concentration of the volatile organic compound returns to the initial level or a steady state level (VOCss) from the corresponding maximum level; and

iv. determining the ventilation rate (k) based on the time taken by the concentration of Volatile organic compound to reach to the initial level from the corresponding maximum level.

In yet further aspects of the present invention, there is provided a system comprising:

a. a compound of interest sensor for providing:

i. a signal indicative of a first concentration of the compound of interest inside the room, wherein the compound of interest is being released by the person

ii. a signal indicative of a second concentration of the compound of interest outside the room;

b. the apparatus for estimating a ventilation rate in the room; and

c. the device for estimating the release rate of a compound of interest.

In yet further aspects of the present invention, there is provided a corresponding computer program which comprises program code means for causing a computer to perform the steps of the methods disclosed herein when said computer program is carried out on a computer as well as a non-transitory computer-readable recording medium that stores therein a computer program product, which, when executed by a processor, causes the method disclosed herein to be performed.

It shall be understood that the apparatus/ device/ system/ computer program product claims will have similar advantages as the method claims.

Preferred embodiments of the invention are defined in the dependent claims. It shall be understood that the claimed method, system, computer program and medium can have similar and/or identical preferred embodiments/ advantage(s) as the claimed method, in particular as defined in the dependent claims and as disclosed herein.

According to a further embodiment, the Volatile Organic Compound (VOC) is released by a person. Release can be in form of flatulence or other physiological processes. According to yet further embodiment, the VOC can be artificially released, such as by releasing perfume from a perfume bottle.

According to a further embodiment, detecting at least one increase from an initial level of the concentration comprises detecting if the increase is rapid, wherein the detecting if the increase is rapid comprises detecting if the increase in concentration is more than a predefined concentration threshold within a pre-defined time threshold.

Advantageously, this helps to understand certain compounds in the room that are temporary in nature, which essentially means that they will also diffuse in sometime and hence understanding the sudden increase and later rate at which they decease help in understanding the ventilation rate of the room.

EP 3 586 739 A1

According to a further embodiment, detecting a corresponding maximum level of the concentration further comprises extracting a peak from the signal indicative of the corresponding maximum level.

According to a further embodiment, determining the ventilation rate comprises extracting a decreasing phase of the signal from the corresponding maximum level to the initial level.

According to a further embodiment, the method further comprises performing the steps of the method over a period of time and averaging the ventilation rate calculated each time.

According to a further embodiment, the release rate is at least one of an exhalation rate, a flatulence rate and rate of release of the compound from skin.

According to a further embodiment, health of the person is analyzed based on the release rate of compound of interest.

The invention can be integrated in solutions related to parenting and child care, e.g., to determine the excretion of compounds indicative of the health or development of a baby. The invention could be integrated in solutions from the sleep monitoring to analyze the health status of a person while the person is sleeping. The invention could be used for home monitoring purposes to track disease/recovery progression of patients or people at risk of certain health conditions. For instance, researchers have identified certain compounds in the breath related to lung and breast cancer (Volatile biomarkers in the breath of women with breast cancer. Phillips M, Cataneo RN, Saunders C, Hope P, Schmitt P, Wai J, J Breath Res. 2010 Jun; 4(2):026003.). Another example is of diabetes in which acetone levels are elevated due to rise of blood sugar level and intensive lipolysis (On the mammalian acetone metabolism: from chemistry to clinical implications. Kalapos MP, Biochim Biophys Acta. 2003 May 2; 1621(2):122-39.). In another example, dysregulation in CO and its levels in exhaled breath has also been implicated in heart disease (Relaxant effects of carbon monoxide compared with nitric oxide in pulmonary and systemic vessels of newborn piglets. Villamor E, Pérez-Vizcaíno F, Cogolludo AL, Conde-Oviedo J, Zaragozá-Arnáez F, Lopez-Lopez JG, Tamargo J Pediatr Res. 2000 Oct; 48(4):546-53.) The invention could perhaps also be used to detect human emitted compounds of interest indicative of poor mouth/teeth hygiene.

In yet further embodiment of the invention the determined ventilation rate can be further used to control an air purifier or air ventilation system. For instance, if it is determined that ventilation rate is a below a predetermined ventilation rate, then the air ventilation system can accordingly be triggered to increase ventilation. Similarity, if the air ventilation is determined below a pre-determined ventilation rate, then the air purification system can be accordingly triggered.

## BRIEF DESCRIPTION OF THE DRAWINGS

These and other aspects of the invention will be apparent from and elucidated with reference to the embodiment(s) described hereinafter. In the following drawings

Fig. 1 shows an overview of various compound of interest (X) present in human breath;

Fig. 2 shows a typical set up a room in which the person, such as a baby, is monitored;

Fig. 3 shows a flowchart detailing the method steps for estimating a ventilation rate in a room;

Fig. 4a shows an example of a VOC signal observed overnight in a baby bedroom;

Fig. 4b shows a zoomed in view of a particular peak in the signal of Fig. 4a;

Fig. 5 shows a flowchart detailing the method steps for estimating a release rate of a compound of interest;

Fig. 6 shows a block diagram of an apparatus for estimating a ventilation rate in a room according to an embodiment of the invention;

Fig. 7 shows a block diagram of a device for estimating release rate of a compound of interest according to an embodiment of the invention; and

Fig. 8 shows a block diagram of a system for estimating release rate of a compound of interest according to another embodiment of the invention.

## DETAILED DESCRIPTION OF THE DRAWINGS

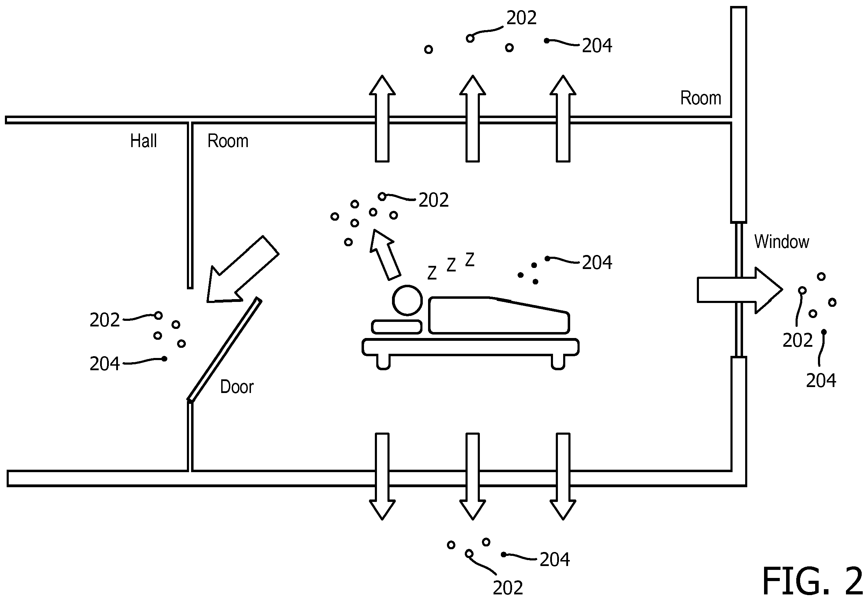

Fig. 2 shows a typical set up a room in which the person is monitored. The person seen in this case is sleeping in the room. It is understood that sleeping is a nice time to monitor a person because there are less disturbances in the room for instance, other people entering the room. To further elaborate, if the health of the baby is to be monitored, then there are high chances that when he/ she is sleeping in the night, there will be less interaction with others, such as parents in the room, and hence the concentration of the compounds in the room will be indicative of the compounds released only by him. In the current figure you can see bigger dots 202 are the compounds of the interest, such as $CO_2$, released by the person by breathing. Further, smaller dots 204 are indicative of Volatile Organic Compounds (VOCs), such as $H_2$, $H_2S$, $CH_3SH$, amines, are released by the person by means of flatulence. As it can be seen that both these compounds diffuse away through means of ventilation (depicted by arrows in the Fig. 2). Thus, it becomes important to understand the ventilation rate of the room in order to understand the release rate (in this case the exhalation rate by means of breath) of the compound of interest in order to further use it to determine the health status.

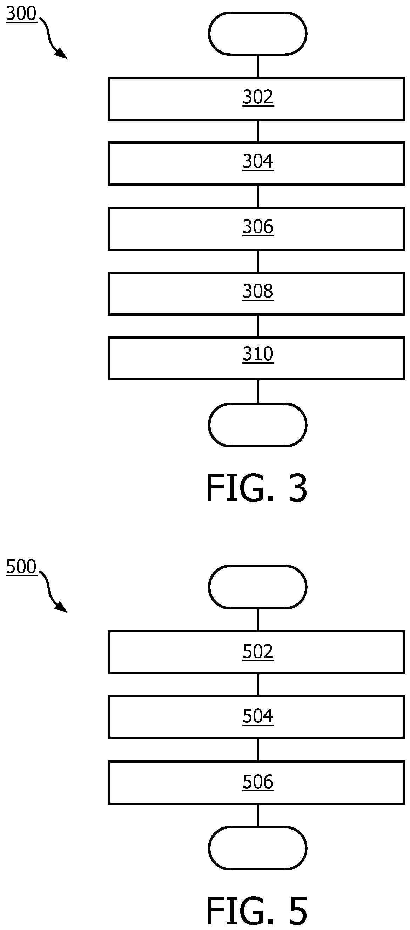

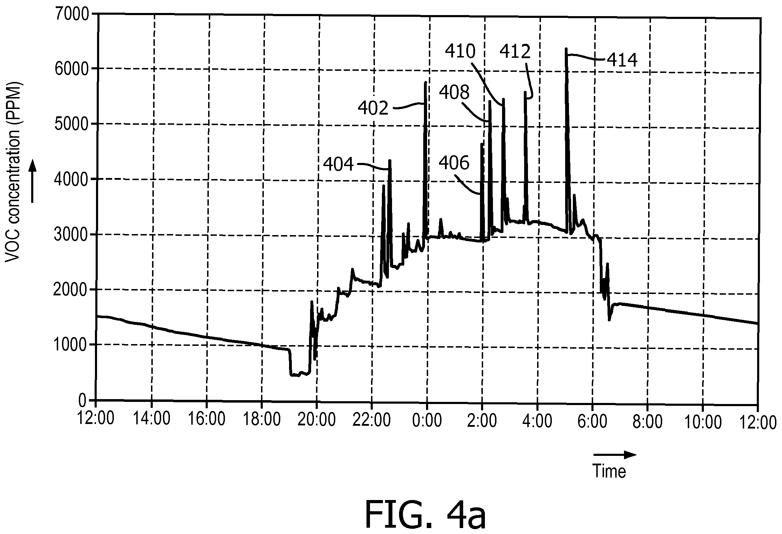

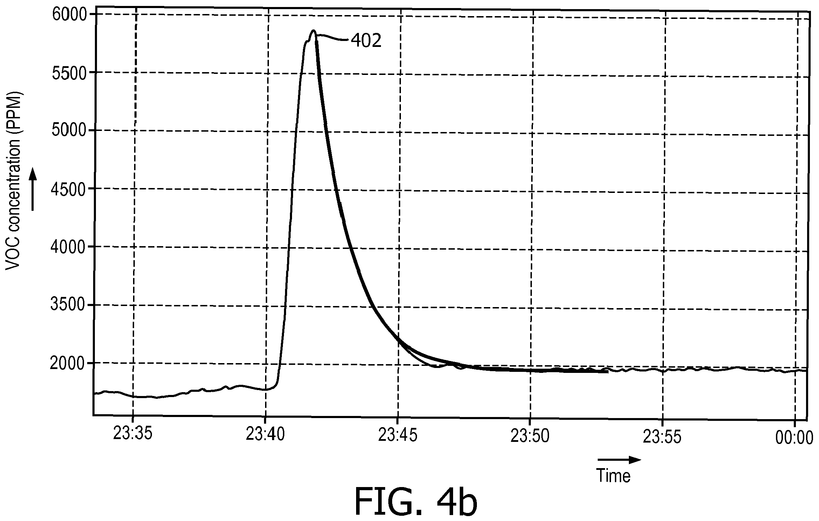

Fig. 3 shows a flow chart (300) detailing the method steps for estimating a ventilation rate in a room. This is explained in conjunction with Fig. 4a and Fig. 4b. Fig. 4a and Fig. 4b show change(s) in concentration of the VOC (y axis) in a time window (x axis). The concentration can be measured/ expressed in ppb/PPB (parts per billion) or ppm/PPM (parts per million). The method begins at step **302** by receiving a signal indicative of a concentration of at least one Volatile organic compound (VOC(t)) inside the room. In an embodiment of the invention a VOC sensor can be installed in the room to monitor a VOC. An example of a VOC sensor is a commercially available AppliedSensor iAQ-2000.

At step **304,** the signal is processed to detect at least one increase from an initial level (VOC(t0)) of the concentration of the at least one volatile organic compound. It is understood that the typical VOC sensor always detects some or the other VOC in the room. Thus, when there is an increase from a current level, it is indicative of release of a certain VOC in the room. In an embodiment, detecting at least one increase from an initial level of the concentration comprises detecting if the increase is rapid. The rapid increase can be further defined as if the increase in concentration of a VOC is more than a predefined concentration threshold within a pre-defined time threshold.

One such increase in the level is detected and a corresponding maximum level from the initial level (i.e. the peak) is detected at **306**. For instance, in Fig, 4a it can be seen there are various increases in the VOC signal, by means of signal peaks (402, 404, 406, ..., 412, 414), observed overnight (1830h to 0630h) in a room of a baby. Thus, one such peak can be peak 402 can be used and extracted for further determination. In an embodiment, the derivative of the VOC signal can be used to extract VOC peaks, such as peak 402. Many of the temporary increases in VOC used for ventilation determination, are related to events in which instantaneously an amount of gas is released (a step-wise response), such as flatulence. Taking into account the diffusion of the gas inside the room, such increases take in the order of seconds to multiple minutes to reach a maximum.

Thereafter, at **308,** it is detected when the concentration of the VOC returns to the initial level or a steady state level (VOC$_{ss}$), i.e. when the peak 402 comes back to the initial level or the steady state level. This is further depicted in Fig. 4b. In an embodiment, the decreasing part of the peak is extracted. Also the derivative can be used for this purpose. The decreasing part of the VOC peak provides information about the room ventilation rate. It may be noted that each of these peaks return to a base level (steady state level) as can be seen in the Fig. 4a and that is due to the ventilation. The faster the VOC level returns to its initial level, the higher the room ventilation rate.

The decrease in the VOC signal, caused by diffusion due to ventilation to the adjacent areas, can be described by the following ordinary differential equation (diffusion model):

$$\frac{d[VOC](t)}{dt} = -k([VOC](t) - [VOC_{ss}]).$$

Here, parameter k represents the room ventilation constant, i.e. the ventilation rate, and [$VOC_{ss}$] is the steady-state level (also may be alternatively referred to as the initial level) the VOC reaches after the decreasing phase.

At step **310,** determining the ventilation rate (k) based on the time (t) taken by the concentration of the at least one Volatile organic compound to reach to the initial level from the corresponding maximum level. In an embodiment, solving the above differential equation gives:

$$[VOC](t) = [VOC_{ss}] - e^{-kt}([VOC_{ss}] - [VOC](t_0))$$

Parameter k is determined/ estimated using a least-squares method that minimizes the sum of squared differences between the simulated VOC level and the measured VOC level. To further elaborate, the diffusion model is fitted using a least-squares technique to estimate the room ventilation rate. The black line in Fig. 4b shows the simulated VOC level.

It may be appreciated that the above procedure can be repeated for each other observed peak, i.e. 404 ... 412, to update the room ventilation rate. Thus, one could also select all identified peaks during a period of time and calculate an average ventilation rate, which will facilitate in a more robust estimation.

Fig. 5 shows a flow chart 500 detailing the method steps for estimating a release rate of a compound of interest, such as compound X. In an embodiment the compound of interest can be $CO_2$. The method described in the Fig. 5 works in conjunction with Fig. 3. The method begins at step **502** by receiving a signal indicative of a first concentration of the compound of interest ([X](t)) inside the room, wherein the compound of interest is being released by the person. In an embodiment, this information is received from a compound of interest sensor, such as $CO_2$ sensor to determine $CO_2$ exhalation. Another example of such a sensor can be a hydrogen sensor. In an embodiment the compound of interest may also be another VOC of interest, such as acetone. In this case the sensor can be an acetone sensor. It may be appreciated that the VOC of interest will be different than the VOC used in Fig. 3 to determine the ventilation rate. The VOC used for determining the ventilation rate are the ones which are temporarily present in the room, for instance by means of flatulence. In an another embodiment, compound of interest sensor can in fact monitor/ detect multiple compounds of interest simultaneously.

At step **504,** a signal indicative of a second concentration of the compound of interest ($X_{out}$) outside the room is received. In an embodiment, this information is also received from a similar sensor as in the step 502, which is placed adjacent, preferably immediately outside, to the room where the person is located/ sleeping.

At **506,** the release rate of the compound ($h_{excr}$) of interest is calculated based on the first concentration, second concentration and the ventilation rate. This is further elaborated below.

In the current embodiment, the release rate is an exhalation rate by means of breathing. A computational model similar to the one used in the method of Fig. 3 is used to reproduce/simulate the measured dynamics of compound X. The model consists of two parts: a part that describes the human exhalation/ excretion of X and a part that describes the diffusion of X to adjacent areas making use of the previously estimated room ventilation. The model is given by the following ordinary differential equation that describes the change in the concentration of X over time:

$$\frac{d[X](t)}{dt} = h_{excr} - k([X](t) - [X_{out}]).$$

Here, $h_{excr}$ represents the human release rate, also may be referred to as human excretion rate of the compound, $[X]$ is the room concentration of the compound, $[X_{out}]$ is the outside concentration of the compound (which in many cases is probably negligible small), and k is the room ventilation rate estimated by means of method steps of Fig. 3. Solving the differential equation gives:

$$[X](t) = \frac{1}{k}\left(h_{excr} + k[X_{out}] - e^{-kt}\left(h_{excr} + k[X_{out}] - k[X](t_0)\right)\right)$$

The parameter of interest $h_{excr}$ is estimated/ calculated using a least-squares method that minimizes the sum of squared differences between the simulated compound level and the measured compound level.

In various embodiment of the invention, once the exhalation rate is determined, which essentially means the exhalation rate of a particular compound of interest, it can be further used to analyze health of the person based on this information. For instance, the compound of interest can be Acetone and Acetone is also known to be elevated in diabetic patients. Thus, understanding the release rate of Acetone can provide insightful analysis of condition of the patient unobtrusively.

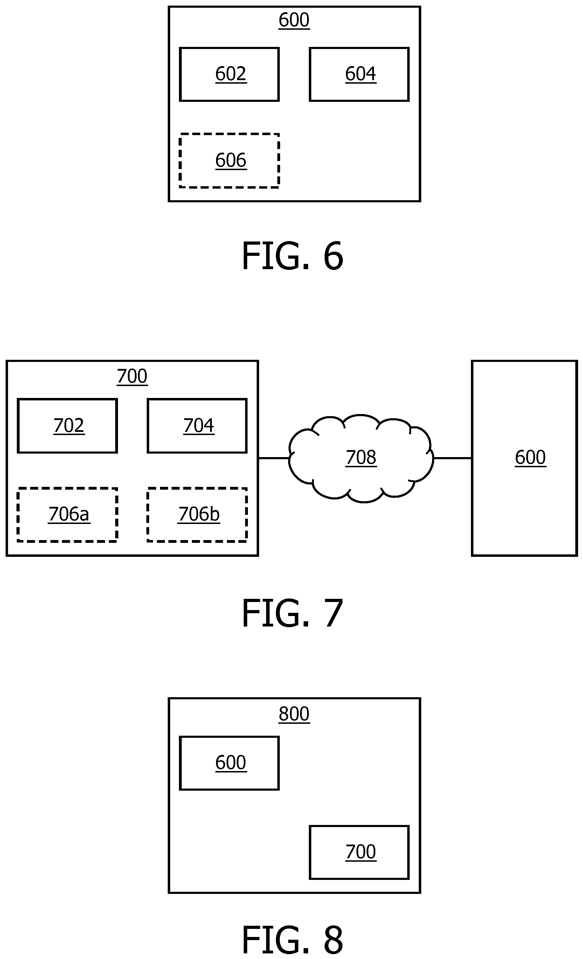

Fig. 6 shows a block diagram of an apparatus **600** for estimating a ventilation rate in a room according to an embodiment of the invention. The apparatus **600** includes a signal interface **602** for receiving a signal indicative of a concentration of at least one Volatile organic compound inside the room, wherein the signal is received from a sensor **606** configured for detecting the at least one Volatile organic compound. In an embodiment of the invention this sensor can be located either within, i.e. integrated in, the apparatus **600** or outside the apparatus **600** (hence depicted by dotted lines) communicating either wirelessly or in a wired manner with the with the signal interface **602.** The apparatus **600** further includes a processing unit **604** to determine ventilation rate of the room. The processing unit **604** process the signal by performing the steps 304 to 310 as explained in Fig. 3.

Fig. 7 shows a block diagram of a device **700** for estimating release rate of a compound of interest according to an embodiment of the invention. The device **700** includes a Compound of interest signal interface module **702** for receiving a signal indicative of a first concentration of the compound of interest inside the room, wherein the compound of interest is being released by the person; and receiving a signal indicative of a second concentration of the compound of interest outside the room. In an embodiment of the invention, the signal indicative of the first concentration and second concentration is received from a first sensor **706a** placed inside the room and a second sensor **706b** placed outside the room. These sensors are of the same type since they are measuring the same compound. These sensors can be part of the same device **700** or can interact either wireless or in the wired manner with the Compound of interest signal interface module **702.** In another embodiment, these sensors can also monitor more than one compound of interest. Once the both signals are received, a processing unit **704** then calculates the release rate ($h_{excr}$) of the compound of interest based on the first concentration, second concentration and the ventilation rate of the room. The calculation of the release rate of the compound of interest has been explained in detail in conjunction with Fig. 5. The ventilation rate required by the device **700** to calculate the release rate ($h_{excr}$) of the compound of interest is provided by the apparatus **600.** In an embodiment of the invention, the device **700** can be in an electronic communication with apparatus **600.** The communication can for instance be wired or wireless, such as network **708.** In another embodiment of the invention, the apparatus **600** can be part of the device **700,** which is also depicted by Fig. 8, where each of the apparatus **600** and device **700** form sub-modules of a system **800.**

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

**1.** A method (300) of estimating a ventilation rate in a room, the method comprising:

receiving a signal indicative of a concentration of at least one Volatile organic compound (VOC(t)) inside the room; processing the signal to

detect at least one increase from an initial level (VOC(t0)) of the concentration of the at least one volatile organic compound;

detect a corresponding maximum level of the concentration from the initial level;

detect when the concentration of the volatile organic compound returns to the initial level or a steady state level (VOCss) from the corresponding maximum level; and

determine the ventilation rate (k) based on the time (t) taken by the concentration of the at least one Volatile organic compound to reach to the initial level from the corresponding maximum level.

**2.** The method according to claim 1, wherein the Volatile Organic Compound is released by a person.

**3.** The method according to any of the preceding claims, wherein detecting at least one increase from an initial level of the concentration comprises detecting if the increase is rapid, wherein the detecting if the increase is rapid comprises detecting if the increase in concentration is more than a predefined concentration threshold within a pre-defined time threshold.

**4.** The method according to any of the preceding claims, wherein detecting the corresponding maximum level of the concentration further comprises extracting a peak from the signal indicative of the corresponding maximum level.

**5.** The method according to any of the preceding claims, wherein determining the ventilation rate comprises extracting a decreasing phase of the signal from the corresponding maximum level to the initial level.

**6.** The method according to any of the preceding claims, wherein the method further comprises performing the steps of the method claims 1-5 over a period of time and averaging the ventilation rate calculated from performing the method claim 1-5 each time.

**7.** The method according to any of the preceding claims further comprising estimating release rate of a compound of interest, wherein the release rate is of the person, wherein estimating the release rate (500) further comprises:

a. receiving a signal indicative of a first concentration of the compound of interest ([X](t)) inside the room, wherein the compound of interest is being released by the person;

b. receiving a signal indicative of a second concentration of the compound of interest ($X_{out}$) outside the room; and

c. calculating the release rate of the compound ($h_{excr}$) of interest based on the first concentration, second concentration and the ventilation rate.

8. The method according to claim 7, wherein the release rate is at least one of an exhalation rate, a flatulence rate and rate of release of the compound from skin.

9. The method according to claim 7 or 8 further comprising analyzing health of the person based on the release rate of compound of interest.

10. The method according to any of the preceding claims, wherein the person is a baby.

11. An apparatus (600) for estimating a ventilation rate in a room, the apparatus comprising:

   a. a signal interface (602) configured for receiving a signal indicative of a concentration of at least one Volatile organic compound inside the room, wherein the signal is received from a sensor (606) configured for detecting the at least one Volatile organic compound;
   b. a processing unit (604) configured for:

      i. detecting at least one increase from an initial level of the concentration of the at least one volatile organic compound;
      ii. detecting a corresponding maximum level of the concentration from the initial level;
      iii. detecting when the concentration of the volatile organic compound returns to the initial level or a steady state level ($VOC_{ss}$) from the corresponding maximum level; and
      iv. determining the ventilation rate (k) based on the time taken by the concentration of Volatile organic compound to reach to the initial level from the corresponding maximum level.

12. The apparatus according to claim 11 further comprising a Volatile Organic Compound sensor (606) for providing a signal indicative of a concentration of at least one Volatile organic compound inside a room.

13. A device (700) for estimating release rate of a compound of interest, wherein the release rate is of the person, the system comprising:

   a. a Compound of interest signal interface module (702) for:

      i. receiving a signal indicative of a first concentration of the compound of interest inside the room, wherein the compound of interest is being released by the person;
      ii. receiving a signal indicative of a second concentration of the compound of interest outside the room; and

   b. a processing unit (704) configured for calculating the release rate ($h_{excr}$) of the compound of interest based on the first concentration, second concentration and the ventilation rate, wherein the ventilation rate is provided by the apparatus of claim 11.

14. A system comprising:

   a. a first compound of interest sensor (706a) for providing a signal indicative of a first concentration of the compound of interest inside the room, wherein the compound of interest is being released by the person
   b. a second compound of interest sensor (706b) for providing a signal indicative of a second concentration of the compound of interest outside the room;
   c. the apparatus (600) for estimating a ventilation rate in a room according to claim 12; and
   d. the device (700) according to claim 13.

15. A computer program product comprising computer program code means which is adapted, when run on a computer, to receive a signal indicative of a concentration of at least one Volatile organic compound inside a room and to perform the steps of processing of the method of any one of claims 1 to 6.

FIG. 1

EP 3 586 739 A1

FIG. 2

300

302

304

306

308

310

# FIG. 3

500

502

504

506

# FIG. 5

FIG. 4a

FIG. 4b

FIG. 6

FIG. 7

FIG. 8

Europäisches
Patentamt

European
Patent Office

Office européen
des brevets

**PARTIAL EUROPEAN SEARCH REPORT**

Application Number

under Rule 62a and/or 63 of the European Patent Convention.
This report shall be considered, for the purposes of
subsequent proceedings, as the European search report

EP 18 18 0023

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HARB P ET AL: "The 40m3Innovative experimental Room for INdoor Air studies (IRINA): Development and validations", CHEMICAL ENGINEERING JOURNAL,, vol. 306, 28 July 2016 (2016-07-28), pages 568-578, XP029757914, ISSN: 1385-8947, DOI: 10.1016/J.CEJ.2016.07.102 | 1-7, 9-12,14, 15 | INV. A61B5/08 G01N33/00 ADD. A61B5/145 F24F11/00 |
| A | * sections 1., 3.2., 3.3., 4.4., 4.6.1., 4.6.3.; figures 1,8,10 * | 8 | |
| X | PEI JINGJING ET AL: "Long-term indoor gas pollutant monitor of new dormitories with natural ventilation", ENERGY AND BUILDINGS, LAUSANNE, CH, vol. 129, 9 August 2016 (2016-08-09), pages 514-523, XP029716398, ISSN: 0378-7788, DOI: 10.1016/J.ENBUILD.2016.08.033 | 1-7, 9-12,14, 15 | |
| A | * sections 1., 2.2., 2.3.2., 2.4., 3.1.; figure 3 * | 8 | TECHNICAL FIELDS SEARCHED (IPC) A61B G01N |

-/--

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 1 February 2019 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

page 1 of 3

**PARTIAL EUROPEAN SEARCH REPORT**

Application Number

EP 18 18 0023

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | M. Hori ET AL: "Measurement of ventilation rate by concentration decay method with law-environmental-loading tracer gas", Proceeding of the 10th International Conference on Indoor Air Quality and Climate - 4-9 September 2005, Beijing, China., 4 September 2005 (2005-09-04), pages 3338-3342, XP55550571, Retrieved from the Internet: URL:https://www.isiaq.org/docs/PDFs/3338.pdf [retrieved on 2019-02-01] | 1-7, 9-12,14, 15 | |
| A | * Sections "MATERIAL AND METHODS", "Comparison of isobutene and sulfur hexafluoride" and "Procedures of measurement" * | 8 | **TECHNICAL FIELDS SEARCHED** (IPC) |
| A | WO 2017/046103 A1 (KONINKLIJKE PHILIPS NV [NL]) 23 March 2017 (2017-03-23) * page 5, line 8 - page 11, line 15; figures 1,2 * | 1-12,14, 15 | |
| A | Detlef Laussmann ET AL: "Air Change Measurements Using Tracer Gases", Robert Koch Institute Germany , 27 July 2011 (2011-07-27), XP55549036, DOI: 10.5772/18600 Retrieved from the Internet: URL:https://edoc.rki.de/bitstream/handle/176904/973/270A2TGPWvv0.pdf?sequence=1&isAllowed=y [retrieved on 2019-01-29] * the whole document * | 1-12,14, 15 | |

-/--

EPO FORM 1503 03.82 (P04C10)

Europäisches
Patentamt

European
Patent Office

Office européen
des brevets

**PARTIAL EUROPEAN SEARCH REPORT**

Application Number

EP 18 18 0023

| | DOCUMENTS CONSIDERED TO BE RELEVANT | | | CLASSIFICATION OF THE APPLICATION (IPC) |
|---|---|---|---|---|
| Category | Citation of document with indication, where appropriate, of relevant passages | Relevant to claim | | |
| A | C. STÖNNER ET AL: "Real-world volatile organic compound emission rates from seated adults and children for use in indoor air studies", INDOOR AIR, vol. 28, no. 1, 6 July 2017 (2017-07-06), pages 164-172, XP55548933, DK ISSN: 0905-6947, DOI: 10.1111/ina.12405 * the whole document * ----- | 1-12,14, 15 | | |

TECHNICAL FIELDS SEARCHED (IPC)

EPO FORM 1503 03.82 (P04C10)

page 3 of 3

17

Europäisches
Patentamt

European
Patent Office

Office européen
des brevets

**INCOMPLETE SEARCH
SHEET C**

Application Number

EP 18 18 0023

```
Claim(s) completely searchable:
      1-12, 14, 15

Claim(s) not searched:
      13

Reason for the limitation of the search:

1        In the European patent application as filed, the search
division has identified multiple independent claims (apparatus claims 11
and 13) in the same category (apparatus). In accordance with Rule 62a(1)
EPC, the applicant was thus invited to indicate the claims complying with
Rule 43(2) EPC on the basis of which the search is to be carried out.
In his reply the applicant disagreed that claim 11 and claim 13 are
independent claims, arguing that "claim 13 is a dependent claim on claim
11 because claim 13 clearly recites that "the ventilation rate is
provided by the apparatus of claim 11", thereby incorporating the
features of claim 11".
However, it is not agreed with this argument, because a dependent claim
is defined as a claim which includes all features of an other claim (Rule
43(4) EPC), wherefore claim 13 is an independent claim, because it does
not include all features of claim 11.The reason is that the feature of
claim 13, that "the ventilation rate is provided by the apparatus of
claim 11", does not imply that the apparatus of claim 11 is part of the
device of claim 13. It is only in claim 14 that the subject-matter of
both, claims 11 and 13, is included.
Therefore the objection under Rule 43(2) EPC is maintained and, given
that the applicant did not indicate which of said independent claims is
to be searched, the search report is issued with the search conducted
based on the first independent claim in the respective category
(apparatus), i.e. claim 11 (see also Guidelines B-VIII, 4.2.2). The
search was therefore restricted to claims 1-12, 14 and 15.
```

## ANNEX TO THE EUROPEAN SEARCH REPORT
## ON EUROPEAN PATENT APPLICATION NO.

EP 18 18 0023

This annex lists the patent family members relating to the patent documents cited in the above-mentioned European search report.
The members are as contained in the European Patent Office EDP file on
The European Patent Office is in no way liable for these particulars which are merely given for the purpose of information.

01-02-2019

| Patent document cited in search report | Publication date | Patent family member(s) | Publication date |
|---|---|---|---|
| WO 2017046103    A1 | 23-03-2017 | CN      108024897  A<br>EP        3349710  A1<br>JP    2018534955  A<br>US    2018235513  A1<br>WO    2017046103  A1 | 11-05-2018<br>25-07-2018<br>29-11-2018<br>23-08-2018<br>23-03-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AGAPIOU et al.** Trace detection of endogenous human volatile organic compounds for search, rescue and emergency applications. *Trends in Analytical Chemistry,* 2015, vol. 66, 158-175
- **PHILLIPS M ; CATANEO RN ; SAUNDERS C ; HOPE P ; SCHMITT P ; WAI J.** Volatile biomarkers in the breath of women with breast cancer. *J Breath Res.,* June 2010, vol. 4 (2), 026003

- **KALAPOS MP.** On the mammalian acetone metabolism: from chemistry to clinical implications. *Biochim Biophys Acta.,* 02 May 2003, vol. 1621 (2), 122-39
- **VILLAMOR E ; PÉREZ-VIZCAÍNO F ; COGOLLUDO AL ; CONDE-OVIEDO J ; ZARAGOZÁ-ARNÁEZ F ; LOPEZ-LOPEZ JG ; TAMARGO J.** Relaxant effects of carbon monoxide compared with nitric oxide in pulmonary and systemic vessels of newborn piglets. *Pediatr Res.,* October 2000, vol. 48 (4), 546-53